# EUROPEAN PATENT APPLICATION

(11) **EP 1 905 362 A1**
(43) Date of publication of application: **02.04.2008**
(21) Application number: 07002902.0
(22) Date of filing: 12.02.2007
(51) Int. Cl.: A61B 17/16, A61B 17/32, A61B 17/00

(54) **Precision assembleable surgical tool handle with limited-play interconnect mechanism**

(30) Priority: 29.09.2006 US 536792
(71) Applicant: PRECIMED S.A., 2534 Orvin (CH)
(72) Inventor: Lechot, André, 2534 Orvin (CH)
(74) Representative: Mötteli-Mantelli, Novella

(57) **Abstract**

A precision adjustable surgical tool holder/driver is provided which is easily disassembled for cleaning and precisely reassembled. The holder/driver has a positionable handle, allowing adjustment of the position of the handle about the spindle of the holder to enable the "handedness" of the holder to be changed in order to accommodate a user while operating from the left or right side of the patient, standing behind or in-front of the patient, or for use in different surgical methods. The holder/driver includes a "limited-play" capture mechanism, which connects the drive end of the housing to its locking sleeve via a limited-play locking device. The limited-play locking device utilizes a disengageable, precision fitted bayonet pin and seat combination in part to accomplish the precision reassembly feature of the present invention.

## Description

### Field of the Invention

The present invention is in the field of surgical tools and accessories for performing orthopedic surgery. More particularly, the present invention relates to handles-tool holders useful in orthopedic surgical procedures, the handles/holders being precision surgical tool handles with a limited-play interconnect mechanism.

### Background

Complicated mechanical devices have crevices and recesses that are difficult, if not almost impossible, to clean with ease. Devices that are not properly cleaned and sterilized contribute to the risk of disease transfer from patient to patient following the emergence of certain "prions" that are not killed by normal hospital sterilization and need to be physically removed by washing/rinsing. Although surgical tool handles are known in the field (see PCT/GB2002/02934 to Chana, incorporated herein by reference), those using "J-slot" (bayonet type) interconnect mechanisms can be subject to play at the interconnections. It would be beneficial in the field to have an interconnect mechanism that has the advantages of the existing "J-slot" interconnect mechanisms, but is less subject to play in the connection.

### Summary

The present invention comprises an adjustable reamer spindle designed to aid a surgeon to better control the instrument. Adjustment of the position of the handle axis of the spindle enables the axis through the palm/grip of each hand to change in order to provide maximum control in different orientations. The adjustment is desirable in order to accommodate operating on the left or right side of the patient, standing behind or in front of the patient, or the use of a different surgical approach. Further, adjustment is important to accommodate the differing needs of surgeons who are naturally left or right handed. In such an instrument that is intended for either right or left handed use, or both CW and CCW rotation in use, it is important to provide an interconnect mechanism that minimizes axial play when used with either hand, or both CW and CCW rotation.

The present invention comprises a precision surgical tool holder/handle which is assemblable over and over again to precise radial, axial and length relationships of its components. The precision tool driver has a precision spindle housing in which a drive train is retained. The drive chain has a drive attachment end connectable to a means for rotating the drive chain, and a tool holder end connectable to a surgical tool head. The tool holder end has an axis of rotation relative to the spindle housing, and an axial displacement aspect relative to a tool end of the spindle housing. The axis of rotation is precisely defined by the relationship between the spindle housing and the drive chain at the tool holder end of the drive chain. The axial displacement aspect is definable as the distance between the tool end of the spindle housing and the tool holder end.

The handle has a fitted locking sleeve which closely receives the spindle housing and drive chain combination. The locking sleeve has a sleeve axis which is disposed precisely parallel to the axis of rotation when the spindle housing and drive chain combination is received by the locking sleeve. The locking sleeve has a mating means interfacing with the spindle housing which precisely fixes a radial aspect relationship between the spindle housing and the locking sleeve when the spindle housing is received by the locking sleeve.

A precision locking device is retained on the locking sleeve. The locking device has an annular collar slideable on the drive end of the locking sleeve, between a sleeve shoulder and a collar ring. The annular collar has a handle attached to it, a collar axis, and an attachment point at which the handle is fixed to the annular collar. Typically, the attachment point is disposed on a radius of the collar axis. The collar ring is releaseably engageable to bias the collar against the sleeve shoulder at a precision radial interface to precisely fix the radial aspect relationship between the attachment point and the collar radius.

A releaseable capture mechanism is provided to integrate the assemblies of the present invention into a working whole. The capture mechanism is embodied in part in each of: the spindle housing/drive chain, locking sleeve and locking device assemblies. The capture mechanism comprises a precision bayonet-type connection cooperatively involving the spindle housing, the locking sleeve and the locking device. The precision bayonet connection includes the collar ring which has an internal surface from which at least two bayonet pins extend radially inward. The bayonet pins pass through retainer slots disposed in the locking sleeve, as described elsewhere. When the locking sleeve assembly is slid over the spindle housing/drive chain assembly, the bayonet pins are received into bayonet slots on the spindle housing corresponding to the retainer slots on the locking sleeve. The bayonet slots are disposed to releaseably engage the bayonet pins. At least one of the bayonet slots has a precision pin seat to engage a precision bayonet pin head on its corresponding bayonet pin.

When the capture mechanism is engaged, the spindle housing/drive chain assembly, the fitted locking sleeve assembly and the locking device all cooperate through the capture mechanism to provide the present precision surgical tool driver repeatably assembleable to precise radial, axial and length aspect relationships of its components and the device overall.

The releaseable capture mechanism has a precision bayonet-type connection between the spindle housing, the locking sleeve and the locking device. The collar ring has an internal surface from which at least two bayonet pins extend radially inward, and pass through retainer slots disposed in the end of the locking sleeve. The retainer slots correspond to bayonet slots in the spindle housing. The bayonet slots are disposed to releaseably engage the bayonet pins. At least one of the bayonet slots has a precision pin seat to engaging a precision bayonet pin head on its corresponding precision bayonet pin.

The spindle housing and drive chain combination, the fitted locking sleeve, the locking device and the capture mechanism all cooperating to provide the present repeatably assembleable precision surgical tool driver.

### Brief Description of the Drawings

FIG. 1 is a side view of the reamer spindle of the present invention.
FIG. 2 is a top view of the reamer spindle of the present invention.
FIG. 3 is a section view taken along line 3-3 shown in FIG. 2.
FIG. 4 is a plan view showing a traditional reamer spindle of the prior art being used in a minimally invasive approach for reaming the acetabular socket.
FIG. 5 is a plan view showing the reamer spindle of the present invention being used in a minimally invasive approach for reaming the acetabular socket.
FIG. 6 is an exploded assembly of an alternative embodiment of the present invention.
FIG. 7 is an alternative shape housing of the alternative embodiment shown in FIG. 6.
FIG. 8 is a perspective view of an alternate embodiment of the invention having a repositionable handle.
FIG. 9A is an exploded view of the alternate embodiment of FIG. 8.
FIG. 9B is a close up of a portion of the exploded view of the alternate embodiment of FIG. 8.
FIG. 9C is a cross-sectional view of the adjustable handle portion of the alternate embodiment of FIG. 8.
FIG. 9D is a perspective drawing of the principle assemblies of the present invention illustrating their axial, radial and length aspects wherein the precision features of the device reside.
FIG. 10 is a perspective view of key components of the alternate embodiment of FIG. 8.
FIGS. 11A and 11B are end-on views of the drive ends of the spindle housing and the locking sleeve assembly, illustrating a limited play interconnection between the housing and the sleeve, with the fitted bayonet pin disengaged from its seat (A), and engaged in its seat (B).
FIG. 11C is an alternative embodiment of the fitted bayonet pin and seat features of the limited-play interconnection of FIGS. 11A and 11B.
FIG. 12 is a perspective end view of the spindle housing showing the bayonet pin J-slots, one of which includes a close tolerance, fitted bayonet pin seat.
FIGS. 13A and 13B are instructional illustrations of a manner in which the locking device can be operated.
FIG. 14 is a plan view of a surgical reamer kit of the invention.

### Detailed Description of the Invention

Referring now to the drawings, the details of embodiments of the present invention are graphically and schematically illustrated. Like elements in the drawings are represented by like numbers, and any similar elements are represented by like numbers with a different lower case letter suffix.

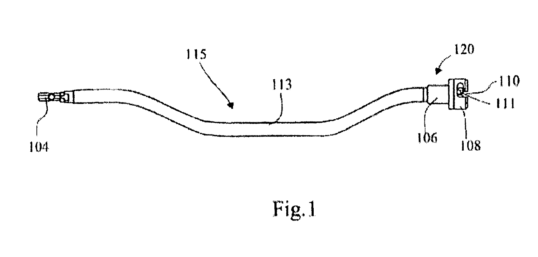

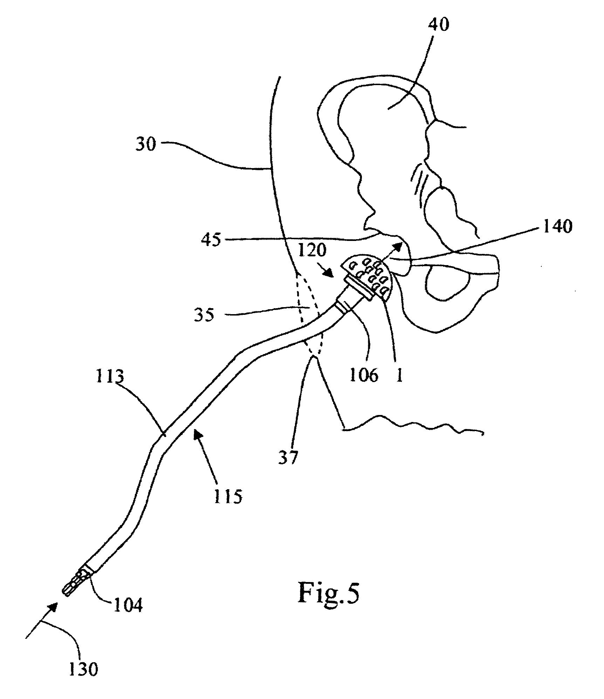

In one embodiment, the present invention comprises a reamer spindle 115 as shown in FIGS. 1-3, and 5. The reamer spindle 115 has a housing 113 containing a drive shaft 107. The drive shaft 107 has a proximal end drive fitting 104 adapted to be attachable to a controllable motive source (not shown). The motive source is provided to rotate the drive shaft 107 inside the reamer spindle 115. The drive shaft 107 has a distal end tool holder fitting 120 for holding a tool head 10, such as a reamer in the embodiment illustrated (see FIG. 5). The drive fitting 104 and the tool holding fitting 120 are disposed to mate with a complimentary fitting on a motive source and a tool head 10, respectively.

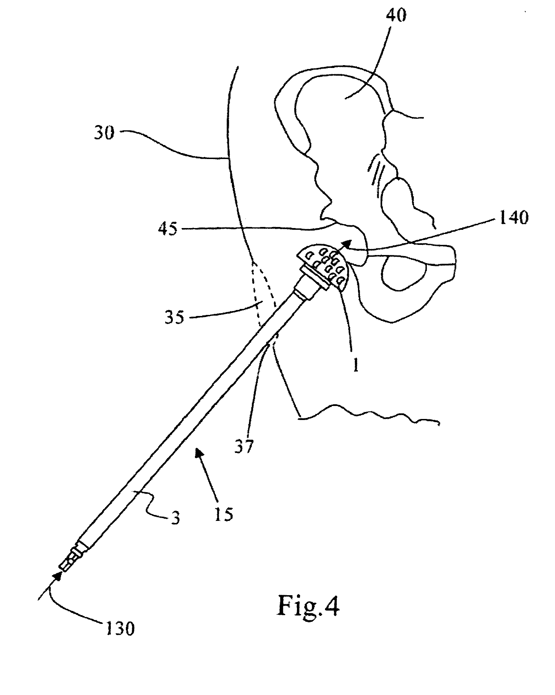

In the embodiments illustrated, the tool holding fitting 120 is illustrated as comprising a complementary part of a bayonet-type connection mechanism. However, as further illustrated below, a complementary part of a bayonet-type mechanism can be used at other connections on a tool spindle 115. The tool holding fitting 120 had a slide 106 carrying a pin 111. The pin 111 works cooperatively with the catch 110 located in the head 108 to form the bayonet for capturing the complementary fitting on a tool head 10, while allowing easy release. The tool heads 10 (reamers in this embodiment) selected for use with the reamer spindle 115 can be shaped and sized for cutting different osseous sites within the body. It is widely known that reamers can be designed to cut the patella in a knee or the glenoid in a shoulder or the socket 45 in an acetabulum 40 as shown in FIGS. 4 and 5.

Referring to FIGS. 4 and 5, the reamer spindle 115 of the present invention and the spindle 15 of the prior art invention are shown passing through a miniature incision 35 in the patient's skin 30. In prior art FIG. 4, the reamer spindle 15 is shown approaching the acetabulum 40 in a preferred orientation for reaming the socket 45. A difficulty with the prior art spindle 15 is that, as shown, the shaft 3 can impinge on the miniature incision 35 at edge 37 of the incision. The current surgical protocols are being pushed to the limits and the incision sizes are being reduced with the intent of increasing the patient's recovery speed. In some cases, surgeons are using a two-incision approach, one to reach the acetabulum and the other to reach the femur. Depending on the situation, either the one incision or the two incision technique can result in less trauma to the patient, thus requiring the instruments to be flexible and more optimally designed to make up for the lack of operating space.

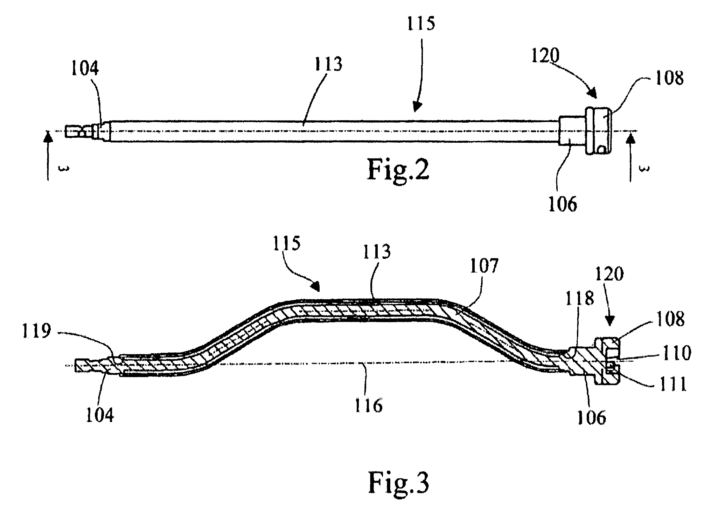

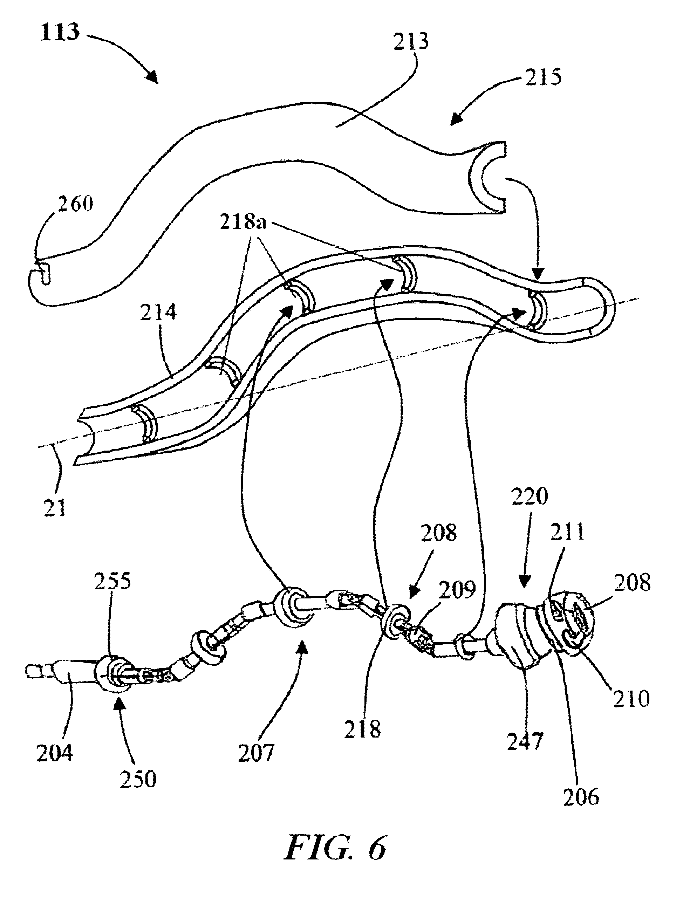

The reamer 115 of FIG. 3 shows a present reamer spindle 115, which has a bent housing 113 containing the drive shaft 107. The drive shaft 107 can be selected from a variety of current torque transmitting mechanisms or devices including a Nickel Titanium shaft, a flexible round or flat wire wound cable, a series of gear driven shafts, or a series of shafts interconnected by universal joints. The drive shaft 107 can also be selected from any torque transmission mechanism or device deemed appropriate for the application, as selectable by one of ordinary skill in the art for practice in the present invention. As illustrated, the drive shaft 107 can be held to the housing 113 with an optional series of bearing surfaces 118 &119 which keep the drive shaft 107 from bearing against/riding on the inside of the housing 113, and can act as a shield to protect the inner housing from blood. Other means for holding the shaft to the housing would be acceptable. The most important feature of the drive shaft 107 is that it conforms to the selected housing 113 and sufficiently supplies torque to the tool head 10. In these examples, the housing 113 is hollow and maintains the drive end fitting 104 substantially collinear with the tool holder fitting 120 (see FIGS. 3 and 6), but as illustrated below, other configurations are intended as well.

Referring now to FIG. 6, an alternative embodiment is shown. Similar to FIGS. 1-3 and 5, the reamer spindle 215 has a housing 113 in two parts 213 & 214 containing a drive shaft 207. The drive shaft 207 has a proximal end drive fitting 204 adapted to be attachable to a controllable motive source (not shown). The motive source is provided to rotate the drive shaft 207 inside the reamer spindle 215. The drive shaft 207 has a distal end tool holder fitting 220 for holding a tool head 10. The drive fitting 204 and the tool holding fitting 220 are disposed to mate with a complementary fitting on a motive source and a tool head 10, respectively. The drive shaft 207 can be selected from a variety of current torque transmitting mechanisms or devices including a Nickel Titanium shaft, a flexible round or flat wire wound cable shaft, a series of gear driven shafts, or a series of linkages 208 interconnected by universal joints 209. The drive shaft 207 can also be selected from any torque transmission mechanism or device deemed appropriate for the application. In this embodiment, the shaft 207 is constructed from a series of linkages 208 containing universal joints 209 and bearing members 218 which rest against journey supports 218a in the housing parts 213 and 214.

The drive shaft 207 can be flexible substantially through out its length, but is required to be flexible only along portions of its length received in curved portions of the housing 113, since it is not necessary to feed drive shaft 207 into the housing 113. Along straight portions of its length, the drive shaft 207 can be rigid. Flexibility in drive shaft 207 is required only to allow its rotation within curved portions of the housing 113. As noted above, the drive shaft 207 can be conventional wound-wire cable flexible along its length, and having one or more alternating layers wound in opposite directions. A protective, friction reducing sheath (not shown) can be provided on such a drive shaft. Other types of flexible drive shafts also can be used. Spaced bearing journals (not shown) and/or lubricant can be provided within outer shaft 50, to allow proper position of drive shaft 56 in outer shaft 50, and to reduce resistance to rotation of drive shaft 56 within outer shaft 50.

The tool holder fitting 220 preferably comprise a complementary part of a bayonet-type mechanism with a slide 206 carrying a pin component 211. The pin 211 works cooperatively with the catch 210 located in the head 208 to form the bayonet for capturing different size reamers while allowing their easy release for size interchangeability and cleaning. The drive shaft 207 is set in housing parts 213 & 214, which are separable for cleaning.

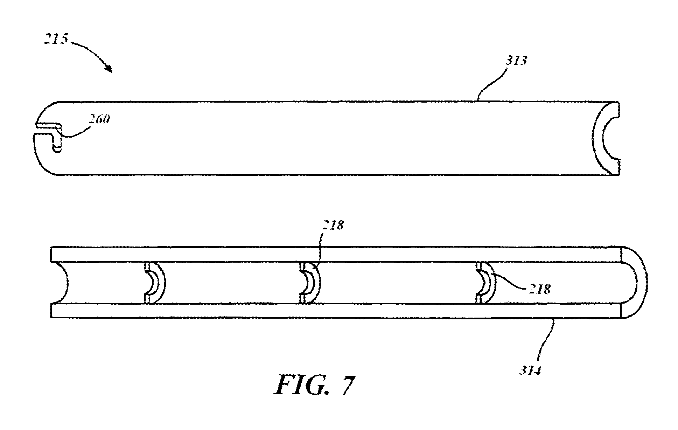

There are many ways of connecting the housing part 213 & 214 together. For example, the drive shaft 207 can include a capture mechanism 247 which is adapted to receive the front ends of the housing parts 213 & 214 aligning each with one another and encapsulating the drive shaft 107 to protect the patient's skin from contacting the torque transmitting shaft 207 during operation. Once the housing parts 213 & 214 are aligned, a locking mechanism 250 comprised of a ring 255 and a catch 260, which is located in the housing member 213, interact with one another to retain the housing parts 213 & 214 in a closed fashion. As with the embodiment described in FIGS. 1-3, and 5, it is preferable to have the drive end 204 substantially collinear with the holding mechanism 220 along axis 216. The housing parts 213 & 214 are shown preferably in FIG. 6 a bent configuration. However, the reamer spindle 215 can embody housing in two parts, wherein the housing parts 313 & 314 are straight and have no bend, as shown in FIG. 7.

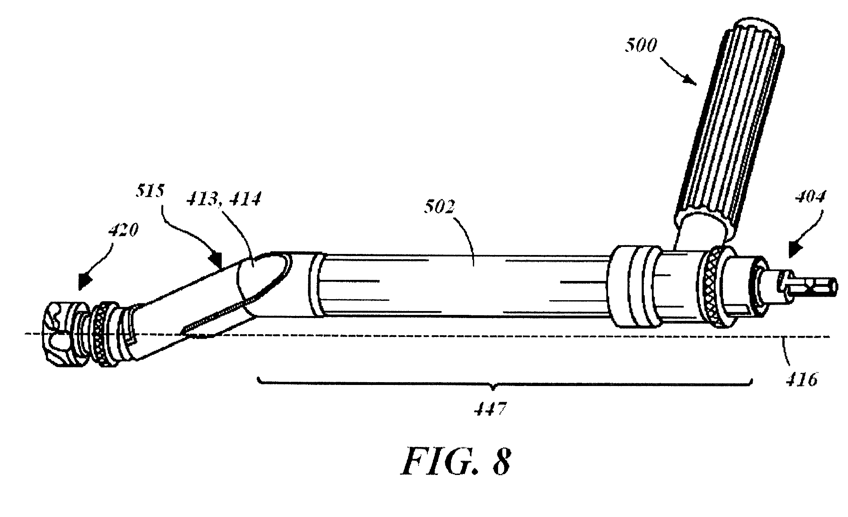

Referring now to FIGS. 8 et seq., alternatively, the drive end 404 is situated along an axis parallel to, but offset from, the axis 416 of the tool holder fitting 420. A bend 480 in the housing is optimally placed at a location to pass through the miniature incision (see FIG. 5) without impinging on the skin 30 at location 37 while still maintaining the same surgical protocol. The drive end fitting 404 and the tool holder fitting 420 have parallel axes, so that an inline force applied to the drive fitting 404 results in an axial force applied to the tool holder fitting 420. This allows the surgeon to maintain the existing surgical technique and accomplish the same result as when a prior reamer spindle 15 is used with its straight drive shaft 3 (see FIG. 4). Thus, the surgeon is able to apply a load directly along the path of reaming.

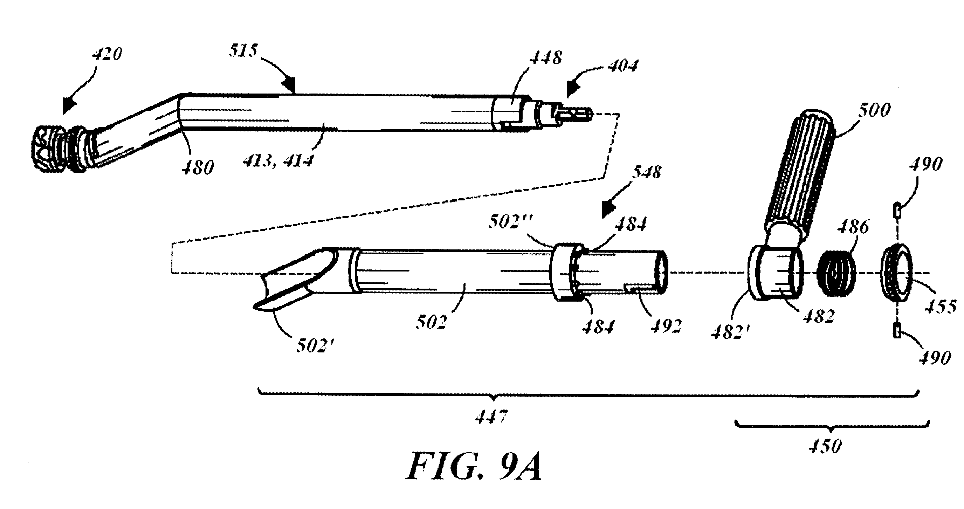

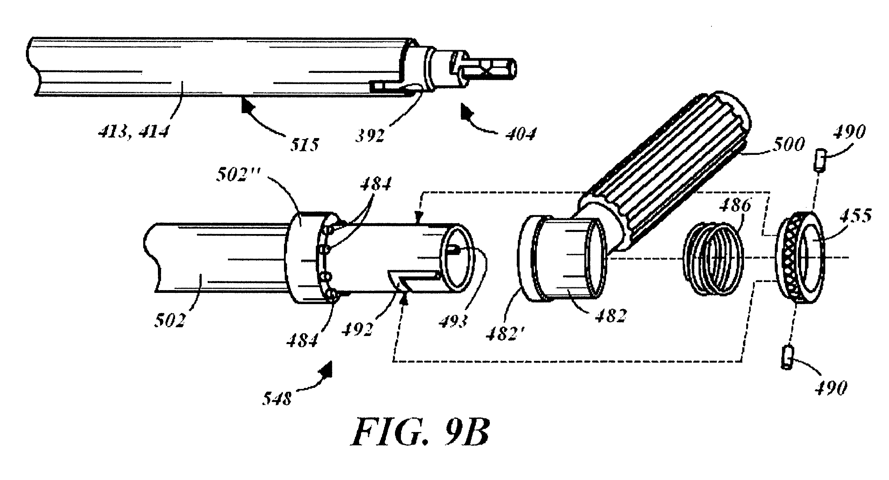

In the embodiment of the reamer spindle 515 illustrated in FIG. 8, the drive end 404 is situated along an axis parallel to, but offset from, the axis 416 of the tool holder fitting 420. Further, as shown in FIGS 9A and 9B, a repositionable handle 500 doubles as a component of the capture device 450 in order to hold the two housing parts 413 & 414 together. The capture mechanism 447 and capture device 450 slide over the front ends 448 of the housing parts 413 & 414, aligning each with one another and thus encapsulating the drive shaft 507 in order to protect the patient's skin from contacting the torque transmitting shaft 507 after the present device is assembled.

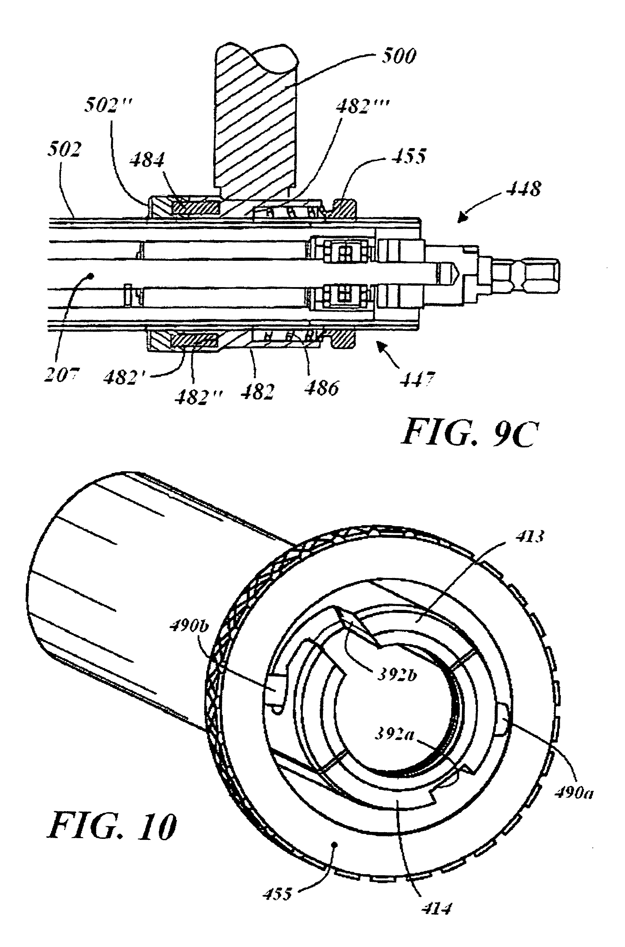

FIGS. 9A, 9B and 9C show how the housing parts 413 & 414 are aligned and locked in place in this embodiment of the spindle 515. The housing parts 413 & 414 are oriented with respect to each other when the locking sleeve 502 (having an internal diameter larger than the outside diameter of the assembled housing 413, 414) slides over them and abuts against a bend 480 in the housing parts 413 & 414. Optionally, a facilitating surface may be disposed between the housing parts 413 & 414 and the locking sleeve 502 to facilitate assembly, such as a thin Teflon® coating or sleeves (not shown). A mouth section 502' of the locking sleeve 502 cradles the bend 480 of the assembled housing parts 413 & 414 and prevents rotation of the locking sleeve relative to the housing parts 413 & 414.

The sleeve drive end 548 of the locking sleeve 502 comprises a capture mechanism 447 which connects the housing drive end 448 of the housing parts 413 & 414 to the locking sleeve 502 via a locking device 450. In one embodiment, the locking device 450 had an annular collar 482 onto which a handle 500 was affixed. The collar 482 includes a face 482' having pin recesses 482" into which sleeve pins 484 are receivable. The sleeve pins 484 are fixed to a sleeve shoulder 502" of the locking sleeve 502. The sleeve pins 484 are disposed to be received into the pin recesses 482". The relationship between the sleeve pins 484 and pin recesses is disposed to provide torsionally rigidly to hold the handle 500 in any one of a number of positions (eight in one embodiment) according to the preference of the surgeon. Alternatively, the sleeve shoulder 502" can have fingers or other projections (not shown) that mate with the recesses 482". A collar spring 486 biases the annular collar 482 into engagement with the sleeve pins 484 by a bias against the annular collar 482. In the embodiment illustrated, this is accomplished by the collar spring 486 applying spring pressure against an internal shoulder 482''' in the annular collar 482 and against a collar locking ring 455. The collar locking ring 455 includes collar pins 490 which are affixed thereto. The collar pins are received in and extend through sleeve bayonet slots 492 in the drive end 548 of the locking sleeve 502. At least one of the sleeve bayonet slots 492 has a close-end 493 (see FIG. 9B), thus retaining the locking device 450, including the collar ring 455 and bias spring 486, on the drive end 548 of the locking sleeve 502, thus keeping the component part of the thus the capture mechanism 447 together when the spindle 515 is disassembled.

In order for the locking device 450 to the engage and connect to the drive end 448 of the assembled housing parts 413 & 414, the collar pins 490 (490a & 490b) extend through the sleeve bayonet slots 492 sufficiently to additionally be received in the housing bayonet slots 392 on the assembled housing parts 413 & 414 (see Fig. 10). As shown in FIG. 10, the housing 413, 414 is held together via the collar pins 490 which engage the bayonet slots 392 in each of the housing parts 413 & 414. The illustration in FIG. 10 has the annular sleeve 502, the collar spring 486 and the locking collar 482 are removed for clarity.

The collar pins 490 of the collar locking ring 455 and the housing bayonet slots 392 interact with one another to retain the housing parts 413 & 414 in an assembled condition, while concurrently biasing the collar spring 486 so as to engage the annular collar 482 (and thus the handle 500) with the sleeve pins 484. Optionally, the bias force of the collar spring 486 can be selected to enable the surgeon to selectively disengage the collar 482 from the sleeve pins 484 and reposition the angle of the handle 500 relative to the locking sleeve 502 in an alternative position, while avoiding disassembly of the spindle 515.

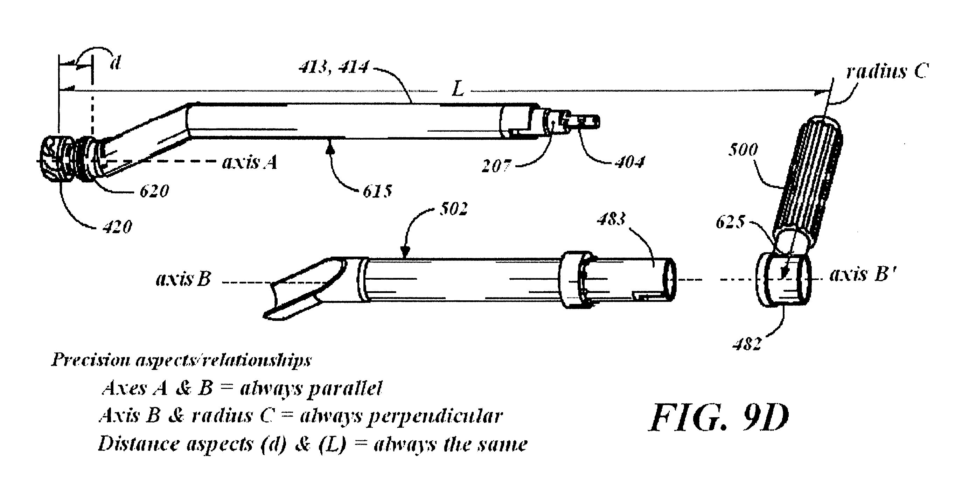

A most important object of the present invention is a precision surgical tool driver which is repeatably assembleable to precise radial, axial and length aspect relationships of its component assemblies. The precision of the device reside in its features which allow it to be disassembled multiple times, and upon each reassembly, the physical dimensions defined by the aspect relationships between its component assemblies are precisely duplicated in the reassembled tool driver. See FIG. 9D. The present precision tool driver comprises three main assemblies and a capture mechanism for precisely interconnecting the main assemblies.

One assembly is a combination spindle housing/drive chain 615. The precision spindle housing 413,414 has an interior disposed to receive and retain the drive train (or drive shaft) 207. The drive chain has a drive attachment end 404 and a tool holder end 420. The drive attachment end 404 is configured to interface with a motive means (not shown) for rotating the drive chain 207. Such motive means are known in the field. The tool holder end is configured to connect to a surgical tool head, such tool holder connector configuration being known in the field. The tool holder end 420 of the drive chain 207 has an axis of rotation aspect A which is parallel relative to the tool end 620 of the spindle housing 413, 414 from which it extends. The precision of the axis of rotation aspect A is defined by the relationship between the spindle housing 413,414 and the drive chain 207 at the tool holder end 420 of the drive chain. More specifically, this relationship is defined by the high precision of the bearing or bushing interface between the spindle housing 413, 414 and the drive chain 207 at the tool end 620 of the housing. This relationship is that the spindle tool end 620 and the tool holder end 420 are always substantially coaxial along the axis of rotation A when the drive chain 207 and the spindle housing 413, 414 are assembled. The tool holder end 420 also has an axial displacement aspect d relative to the tool end 620 of the spindle sleeve. The axial displacement aspect d is the distance between an end point 620 on the spindle housing 413, 414 and the tool holder end 420. The precision of the axial displacement aspect d is defined by the relationship between the spindle housing and the drive chain at the tool holder end of the drive chain, which aspect is substantially always the same when the drive chain 207 and the housing 413, 414 are assembled.

Another assembly is the precision fitted locking sleeve which closely receives the spindle housing and drive chain combination 615. In the embodiment illustrated, the drive end 404 of the spindle housing/drive chain assembly 615 slides into and is closely received by the locking sleeve 502. The locking sleeve 502 has a sleeve axis B. The locking sleeve 502 is configured to precisely receive and retain the spindle housing 413, 414 so that the sleeve axis B is parallel to the axis of rotation aspect A of the tool holder end 420 of the drive chain 207. The locking sleeve 502 also has a mating means 502' which interfaces with the spindle housing 413, 414 to precisely fix the radial aspect of the locking sleeve 502 relative to the spindle housing 413, 414 upon receipt of the spindle housing by the locking sleeve. This is to say, the mating means 502'assures the spindle housing 413, 414 is repeatably receivable in the locking sleeve in the same radial orientation relative to each other. Additionally, in the embodiment illustrated, the mating means 502' provides a precise travel limit on how far the locking sleeve 502 can slide along the spindle housing 413, 414.

The third assembly noted above is the precision locking device 450, which is retained on the locking sleeve 502. The locking device 450 comprises an annular collar 482 slidable on the drive end 483 of the locking sleeve 502 between a sleeve shoulder 502" and a collar ring 455. The collar ring 455 retains the collar 482 on the locking sleeve 502, and incorporates features of a precision bayonet connection, further described elsewhere herein. The collar 482 has a handle 500 attached to it, a collar axis B' through its centerline and a point of attachment 625 of the handle 500 to the collar 482. The collar axis B' is substantially collinear with the sleeve axis **B**, and in the embodiment illustrated, is coaxial. The point of attachment 625 is disposed along a selected radius C of the collar axis. The collar ring 455 is releaseably engageable to bias/hold the collar 482 against the sleeve shoulder 502" at a precision radial interface 484 & 482', a first part 484 of which is on the sleeve shoulder 502" and the second part 482' being on the annular collar 482. The precision radial interface precisely fixes the radial aspect relationship between the point of attachment and the collar axis radius. In the embodiment illustrated, the radial aspect relationship between the point of attachment of the collar axis to the locking sleeve axis B.

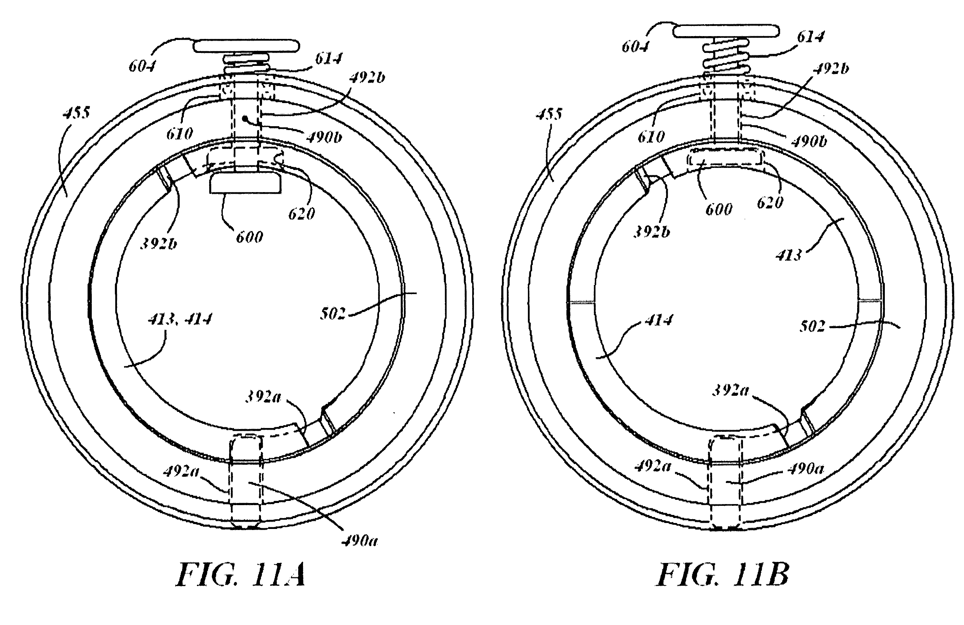

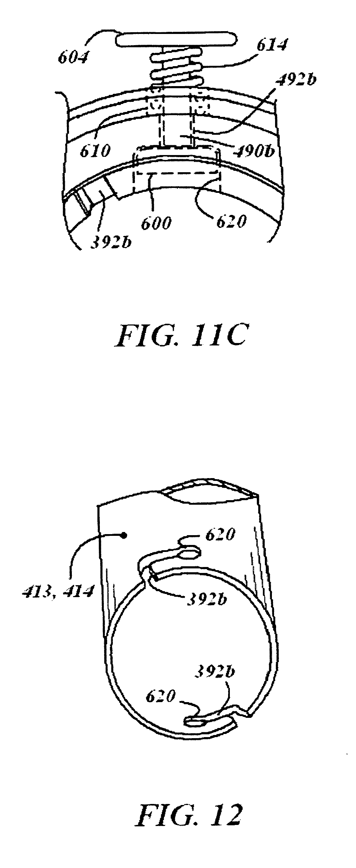

FIGS. 11A - 11C and 12 illustrate the "limited-play" features of the capture mechanism 447, which connects the housing drive end 448 of the housing 413, 414 to the locking sleeve 502 via the locking device 450. FIGS. 11A and 11B are end-on views of the drive ends of the spindle housing and the locking sleeve assembly, with the fitted bayonet pin disengaged from its seat 620 (in FIG. 11A), and engaged in its seat 620 (in FIG. 11B). In the embodiment shown in FIG. 11A, to engage the housing 413, 414, locking device 450 of the capture mechanism 447 has two collar pins 490a & 490b held by the collar ring 455. One is a fixed collar pin 490a and the other is an extendable collar pin 490b. The extendable collar pin 490b has a fitted bayonet pin head 600 at the end of the collar pin 490b disposed in the interior of the collar ring 455, and a push-button cap 604 at the other end of the collar pin 490b disposed anterior to the collar ring 455 and a pin spring 614 disposed about the shaft of the pin 612. The extendable collar pin 490b is slideably received in a pin passage 610 in the collar ring 455. In the embodiment shown, the pin passage 610 also serves a spring chamber for receiving the pin spring 614. The pin spring 614 provides a biasing force against the push-button cap 604 and the collar ring 455, which normally displaces the push-button cap 604 away from the collar ring 455.

The collar pins 490a & 490b are received in and extend through sleeve bayonet slots 492a & 492b in the drive end 548 of the locking sleeve 502 (also see FIG. 10). The collar pins 490a & 490b extend through their respective sleeve bayonet slots 492a & 492b sufficiently be received in their respective housing bayonet slots 392a & 392b on the assembled housing parts 413, 414 (also see Fig. 10). The fitted bayonet slot 390b for receiving the fitted bayonet pin 490b includes a head seat 620. The head seat 620 is disposed to closely receive and securely engage the bayonet pin head 600 of the fitted bayonet pin 290b under the bias force of the pin spring 614. The relationship of the configuration of the pin head 600 and the head seat 620 is disposed to enable the close and securely engagement of the pin head in the head seat. This may be accomplished by any of a number of means known to the ordinary skilled artisan for practice in the present invention, including chamfering or beveling the surface of the head seat 620 to closely receive a complementary surface of the pin head 600.

As shown in FIG. 11B, the housing parts 413 & 414 are held together via the collar pins 490a & 490b which engage their respective bayonet slots 392a & 392b in each of the housing parts 413 & 414. The collar pins 490a & 490b of the collar locking ring 455 and the housing bayonet slots 392a and 392b interact with one another to retain the housing parts 413 & 414 in an assembled condition. Thus, the locking device 450 can engage and connect to the drive end 448 of the assembled housing parts 413 & 414. When the depicted embodiment of the present invention is in its assembled condition, as in FIG. 11B, depressing the push-button cap 604 to over come the bias force causes the pin head 600 to be disengaged from the head seat 620 and to extend beyond the bayonet slot 392b. While the push-button cap is so depressed, the bayonet connection of the locking device 450 can be disengaged in a conventional manner, and the spindle housing 413, 414 can be separated from the capture mechanism 447.

FIG. 11C is an alternative embodiment of the fitted bayonet pin and seat features of the limited-play interconnection of FIGS. 11A and 11B. In this embodiment, head seat 620a comprises both the spindle housing 413, 414 and the locking sleeve 502. This configuration of the pin head seat 620a allows the pin head 600 to be closely receive by and to securely engage with both spindle housing 413, 414 and the locking sleeve 502, to reduce further the possibility of play in the interconnection between the two assemblies.

Its form helps it to lodge itself in the diameter cut at the end of one of the J-slots in the external locking sleeve. This cut diameter is identical to the largest diameter of the mobile pin. The path of the J-slot outside of this specific diameter is sized according to the smaller diameter of the mobile pin. The pin then slides the length of the J-slot and clicks into the diameter cut at the end of one of the J-slots in the external locking sleeve. The corresponding slot in the internal Z sleeve must be cut in order that the large diameter of the mobile pin can slide the entire length of the external J-slot. This slot is in fact an L-slot of which the diameter is that of the larger diameter of the fixed pin.

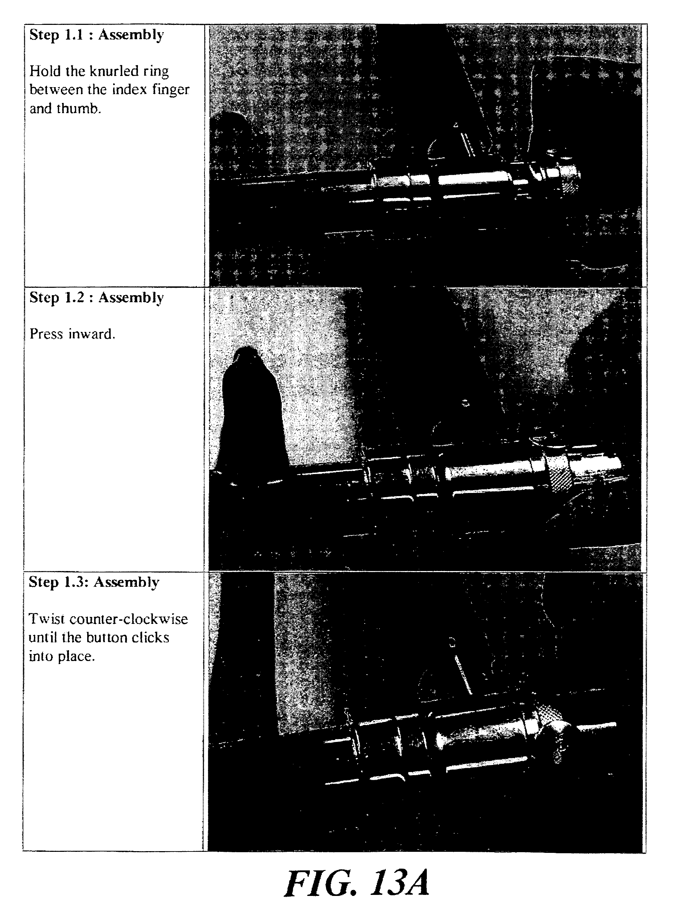

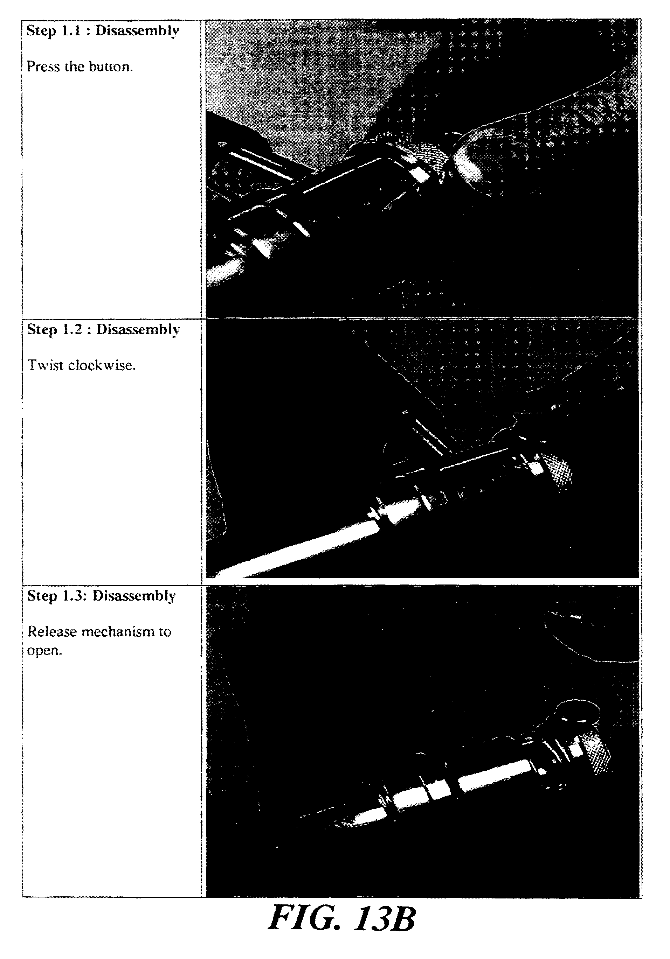

FIG. 12 is a perspective end view of the spindle housing 413, 414 showing the bayonet J-slots 392a & 392b. In some of the embodiments illustrated, one of the two bayonet slots 390 included a close tolerance, fitted bayonet pin seat 620. However, as known to the ordinary skilled artisan, more than two bayonet slots may be practiced in the present invention. Additionally, as shown in FIG. 12, there may be multiple bayonet slots 392b each having a close tolerance, fitted bayonet pin seat 620. FIGS. 13A and 13B are instructional illustrations of a manner in which the locking device can be operated to engage (FIG. 13A) or to disengage (FIG. 13B) the drive end of the spindle housing.

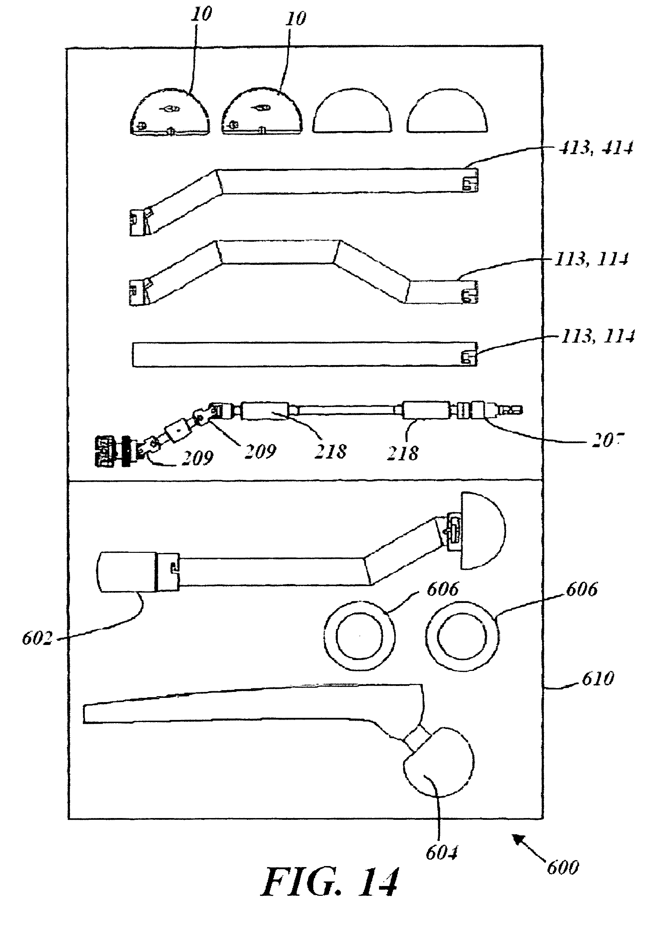

Referring now to FIG. 14, collectively, these different types of housing parts 213-214, 313-314, and 413-414 can be provided as a kit 600 having a selection of different sized reamer housings 113, tool heads 10, an impactor 602, acetabular implants (not shown), femoral hip prostheses 604, and acetabular cup prostheses 606, the selection of different reamer housing configurations allowing the surgeon to select between a bent, offset configuration or a straight configuration of the reamer spindle depending on the surgeons approach, which may vary during the same operation or between different patients.

While the above description contains many specifics, these should not be construed as limitations on the scope of the invention, but rather as exemplifications of one or another preferred embodiment thereof. Other variations are possible, which would be obvious to one skilled in the art. Accordingly, the scope of the invention should be determined by the scope of the appended claims and their equivalents, and not just by the embodiments.

## Claims

1. A precision assembleable surgical tool driver having a radially positionable handle and a limited-play interconnect mechanism comprising:
a drive spindle, the drive spindle having a housing in at least two housing parts separable from one another approximately along a longitudinal axis of the housing, the housing retaining a flexible drive shaft and having a driven end attachment assembly at a proximal end adapted for attachment to a rotary drive source and a driving end attachment assembly at the distal end adapted for selectively engaging and disengaging a surgical tool;
a capture mechanism having a locking sleeve into which the drive spindle is closely receivable for releaseably holding the housing parts in an assembled condition and retaining the drive shaft in the housing; and
a precision locking device disposed at the proximal end of the spindle drive and locking sleeve and cooperating with the capture mechanism and the drive spindle, and having an annular collar slideably disposed on the proximal end of the locking sleeve, to which collar the radially positionable handle is attached, and the locking device disposed to precisely fix the structural relationship between the spindle drive and the locking sleeve by engaging the limited play interconnect mechanism, the limited play mechanism being a precision bayonet-type connection disposed at the proximal end of the spindle drive and locking sleeve, the combination providing the precision assembleable surgical tool driver.

2. The precision assembleable surgical tool driver of claim 1, wherein the precision locking device further comprises a circumferential sleeve shoulder fixed near the proximal end of the locking sleeve, the sleeve shoulder having a first interface part of a precision radial interface, the first interface part engageable with a second interface part disposed on the slideable annular collar, the second interface part normally biased against the first interface part by an elastic device disposed between the annular collar and a locking ring of the limited play interconnect when the locking ring is in an engaged condition, with engagement of the interface parts fixing the positional handle in a radial position relative to the spindle drive.

3. The locking device of claim 2, wherein moving the locking ring to a disengaged condition enables the removal of the locking sleeve from the drive spindle, separation of the housing parts and release of the drive shaft.

4. The precision assembleable surgical tool driver of one of claims 2 or 3, wherein the annular collar has recesses for receiving sleeve projections on the sleeve shoulder fixed to the housing, the annular collar and the adjustable handle being torsionally locked relative to each other when the projections are received into the recesses.

5. The annular collar of claim 4, where in the sleeve projections are sleeve pins.

6. The surgical reamer spindle of any one of claims 2 to 5, wherein the locking ring has at least one bayonet pin affixed thereto, the at least one bayonet pin locking the locking ring in a locking position when the locking ring is biased into a bayonet-type fitting recess by the elastic device.

7. The precision assembleable surgical tool driver of any of the above claims, wherein the drive train is selected from a group of drive trains consisting of nickel titanium drive trains, ferrous metal drive trains, flexible round wound cable drive trains, flat wire wound cable drive trains, gear-driven shaft drive trains, and drive trains having shafts connected via universal joints.

8. A precision surgical tool handle repeatably assembleable to precise radial, axial and length relationships of its components, the tool handle comprising:
a drive spindle, the drive spindle having a spindle housing having a drive end and a tool end and separable into at least two housing parts, a drive train retained in the spindle housing, the drive chain having a drive attachment end connectable to a motive means for rotating the drive chain, and a tool holder end connectable to a surgical tool head, the tool holder end having an axis of rotation aspect relative to the spindle housing and an axial displacement aspect of the tool holder end relative to a tool end of the spindle housing with the axis of rotation aspect being precisely defined by the relationship between the spindle housing and the drive chain at the tool holder end of the drive chain, and the axial displacement aspect being definable as the distance between the tool end of the spindle housing and the tool holder end;
a releaseable capture mechanism having a precision fitted locking sleeve for closely receiving the spindle housing and drive chain combination, the locking sleeve having a sleeve axis, and the sleeve axis being disposed to be precisely parallel to the axis of rotation upon receipt of spindle housing and drive chain combination by the locking sleeve, and the locking sleeve having a mating means interfacing with the spindle housing and precisely fixing a radial aspect relationship between the spindle housing and the locking sleeve upon receipt of the spindle housing by the locking sleeve;
a precision locking device retained on the locking sleeve, the locking device having an annular collar slidable on a drive end of the locking sleeve between a sleeve shoulder and a collar ring, with the annular collar having an attached handle, a collar axis and a point of attachment of the handle to the annular collar, the point of attachment being on a selectable radius of the collar axis, and the collar ring releaseably engageable to bias the annular collar against the sleeve shoulder at a precision radial interface to precisely fix the radial aspect relationship between the point of attachment and the collar radius, and a precision bayonet-type connection mechanism;
the precision bayonet-type connection disposed to mechanically communicate between the spindle housing, the locking sleeve and the locking device, wherein the collar ring has an internal surface from which at least two bayonet pins extend radially inward to pass through retainer slots disposed in a drive end of the locking sleeve, the retainer slots corresponding to bayonet slots in the drive end of the spindle housing, the bayonet slots disposed to releaseably engage the bayonet pins with at least one of the bayonet slots having a precision pin seat to engage a precision bayonet pin head on a corresponding precision bayonet pin; and
the spindle housing and drive chain, the fitted locking sleeve, the locking device and the capture mechanism cooperating to provide the precision surgical tool handle repeatably assembleable to have a precise radial relationship between the point of attachment and the selected radius of the collar axis, a precise axial relationship between the locking sleeve axis and the axis of rotation, and precise length relationships between the handle point of attachment and the spindle housing end and between the tool holder end and the housing end.

9. A releaseable precision locking device for a surgical tool handle having a circular end portion, the releaseable precision locking device comprising: a precision bayonet-type connection between circular end portions of a spindle housing and a locking sleeve of the tool handle, wherein the locking device has a collar ring with an internal surface from which at least two bayonet pins extend radially inward and pass through retainer slots disposed in the circular end of the locking sleeve, the retainer slots corresponding to bayonet slots in the circular end portion of the housing, the bayonet slots disposed to releaseably engage the bayonet pins, with at least one of the bayonet slots having a precision pin seat to engaging a precision bayonet pin head disposed on a corresponding precision bayonet pin.
